# EUROPÄISCHE PATENTANMELDUNG

(11) **EP 1 917 981 A1**
(43) Veröffentlichungstag der Anmeldung: **07.05.2008**
(21) Anmeldenummer: 06022628.9
(22) Anmeldetag: 30.10.2006
(51) Int. Cl.: A61L 9/014, A61L 9/14

(54) **Kombiniertes Adsorptions- und Abgabesystem**

(71) Anmelder: Tex-A-Tec AG, 9630 Wattwil (CH)
(72) Erfinder: Marte, Oliver, 9630 Wattwil (CH); Meyer, Martin, 8800 Thalwil (CH); Zimmermann, Michael, 5033 Buchs (CH)
(74) Vertreter: Forstmeyer, Dietmar

(57) **Zusammenfassung**

Die Erfindung betrifft ein chemisch/mechanisches, sich eigendynamisch regulierendes Dispensersystem für Stoffe, insbesondere für Duftstoffe und/oder Arzneimittel, welches die Wirkstoffe aufgrund des enthaltenen Polymers mit hoher Schmelzenthalpie und ggf. einer niedermolekularen wasserlöslichen Substanz mit endothermer Lösungs- bzw. Sorptionsenthalpie trotz erhöhter Temperatur gleichmäßig abgibt. Zusätzlich gibt die Erfindung die Möglichkeit, die Abgabe von Wirkstoffen bei Überschreiten einer bestimmten Temperatur zu unterbinden.

## Beschreibung

Die Erfindung betrifft ein chemisch/mechanisches, sich eigendynamisch regulierendes Blistersystem für Stoffe, insbesondere für Duftstoffe und/oder Arzneimittel, dessen Anwendung gleichermassen in Wohn- und Arbeitsräumen, Pflegeheimen, und Arztpraxen, aber auch in Autos und Klimaanlagen aller Art sowie in öffentlichen Gebäuden und im Hotel- und Krankenhausbereich möglich ist. Die Erfindung umfasst ferner einen Stoffblister mit einem Wirkstoff sowie einen Druckblister zum Verschliessen des Stoff-dispensersystems. Ferner umfasst die Erfindung einen Blister zur Entfernung von Krankheitserregern, Mikropartikeln und unerwünschten Gerüchen.

Die Zielsetzung, die mit dem Einsatz von Stoffdispensern, insbesondere von Duftdispensem, verbunden ist, ist die Verbreitung von allgemeinem Wohlbefinden. Dies schliesst sowohl die Abgabe von "Wohlgerüchen" bzw. Substanzen, die das allgemeine Wohlbefinden steigern ein als auch die Abgabe von Substanzen zur Eliminierung unerwünschter Gerüche bzw. die Eliminierung unerwünschter Gerüche. Ebenso wichtig ist es, Krankheitserreger, wie z.B. Parasiten, Pilze, Bakterien (Staphylokokken und Escherichia coli) oder Viren, Mikropartikel, wie z.B. Zigarettenrauch, Feinstaub aus Abgasen, sowie unerwünschte gasförmige Substanzen, wie z.B. Schweiß oder Zigarettenrauch, aus der Luft zu entfernen. Die partikulären Substanzen, wie Krankheitserreger oder Mikropartikel, können dabei auch in Form eines Aerosols vorliegen. Während Geschmacksstoffe bezüglich ihres Molekulargewichtes und ihrer Polarität keine Begrenzung kennen, wird die Wahrnehmung von Duftstoffen meist durch relativ kleine, hydrophobe und stark flüchtige Substanzen ausgelöst. Sehr wesentlich ist die Feststellung, dass die Reizauslösung durch Duftstoffe (= Riechstoffe) bereits bei extrem niedrigen Schwellenwerten eintritt, im Gegensatz zu den Geschmacksstoffen (G. Ohloff, Düfte, Verlag Helvetica Chimica Acta AG Zürich, 2004). Durch erhöhte Duftstoffkonzentrationen in der Luft werden die Rezeptormembranen, die als biochemische Detektoren fungieren, für längere Zeit blockiert und anstelle von Wohlbefinden kann Übelkeit und Ekel ausgelöst werden.

### Stand der Technik

Die heute in den verschiedensten Bereichen eingesetzten Duftstoffdispensersysteme zeigen in ihrem Design eine grosse Vielfalt. Häufig anzutreffende Systeme sind Blisterpackungen, die den Duftstoff als Lösung oder mit nicht flüchtigen Chemikalien in verdicktem Zustand, d.h. als Gel, enthalten (z.B. Cinnamon Dream von Migros, Schweiz). Andere Systeme, die häufig in Autos anzutreffen sind, enthalten den Duftstoff als Lösung in einem 5 - 50 ml Speicherbehälter, wobei der Duftstoff z.B. über ein poröses Dochtsystem an die Umgebungsluft abgegeben wird. Zur Stoffabgaberegulierung enthalten diese Systeme vielfach einen mechanischen, von Hand zu betätigenden Verschluss. Weitere, die Stoffabgabe fördernde Systeme enthalten Ventilatoren, bzw. werden in Ventilationskanälen eingebaut. Wiederum andere Duftsysteme bestehen aus einem festen mit Duftstoff imprägnierten Träger, der diesen, ähnlich den eben geschilderten, weitgehend unkontrolliert in die angrenzende Atmosphäre abgibt.

Allen diesen Systemen gemeinsam ist die mehr oder weniger unkontrollierte Stoffabgabe in Abhängigkeit von der Temperatur, d.h. es wird um so mehr Duftstoff abgegeben, je höher die Umgebungstemperatur ist. Dies hat zur Folge, dass in Abhängigkeit der das Duftsystem umgebenden Lufttemperatur hohe Duftstoffabgaberaten erreicht werden, in deren Folge anstelle der "Wellness" verbreitenden Funktion Übelkeit und allergische Reaktionen resultieren. Ein weiterer Nachteil bestehender Systeme ist die temperaturbedingte, viel zu rasche Entleerung des Duftstoffspeichers bzw. Duftstoffbehälters (auch Duftstoffblister oder Stoffblister oder Blister genannt) mit den entsprechenden finanziellen Konsequenzen. Äusserst problematisch wirken sich die erwähnten Nachteile bei sommerlichen Temperaturen aus, da beispielsweise je nach Sonneneinstrahlung in geschlossenen Räumen (z.B. Autos) bis zu 70 °C erreicht werden. Um solchen Extremsituationen zu begegnen, werden typischerweise in Autos mechanisch verschliessbare Duftstoffdispensersysteme eingesetzt. Der wesentliche Nachteil dieser Systeme ist der von Hand zu betätigende und nicht automatisch durch die Umgebungstemperatur kontrollierte Verschlussmechanismus.

Die wichtigsten Hersteller von Wirkstoff-Dispensersystemen sind: Reckitt-Benckiser (Marke: Airwick), Sara Lee (Marke: Ambi Pur), SC Johnson (Marke Glade), Duco Magic Tree, Carfreshener, und Holts.

### Aufgabe und Lösung der Erfindung

Aufgabe der Erfindung ist es, ein automatisch funktionierendes Blistersystem bereitzustellen, das - relativ unabhängig von der jeweiligen Umgebungstemperatur - automatisch stets eine bestimmte, konstante Menge eines Wirkstoffes, wie eines Duftstoffes und/oder Arzneimittels, gleichmässig abgibt.

Eine weitere Aufgabe der Erfindung besteht darin, Krankheitserreger, wie z.B. Parasiten, Pilze, Bakterien (z.B. Staphylokokken und Escherichia coli) oder Viren, Mikropartikel, wie z.B. Zigarettenrauch oder Feinstaub aus Abgasen, sowie unangenehme Gerüche, wie z.B. Schweiß oder Zigarettenrauch, aus der Luft zu entfernen, und gegebenenfalls diese in eine biologisch verträglichere Form zu transformieren.

Als Option soll darüber hinaus bei Überschreiten einer bestimmten Umgebungstemperatur automatisch die Abgabe von Wirkstoffen unterbunden werden, ohne dass es dazu eines menschlichen Eingriffes bedarf.

Diese Aufgaben werden durch Anwendung unterschiedlicher physikalischer, chemischer oder biochemischer Prinzipien gelöst. Zunächst wird die gleichbleibende Abgabe von Wirkstoffen trotz steigender Umgebungstemperatur durch einen im (Wirk-)Stoffblister (d.h. Wirkstoffbehälter) vorhandenen "Temperaturpuffer" aus verschiedenen Substanzen bewirkt, der eine zu starke Aufheizung des Systems verhindert. Zusätzlich wird durch einen automatischen, auf einem thermodynamisch - mechanischen Funktionsprinzip basierenden Verschlussmechanismus die Abgabe von Wirkstoffen bei Überschreiten einer bestimmten Umgebungstemperatur durch einen Druckblister (d.h. Druckbehälter) unterbunden.

Beide Mechanismen der vorliegenden Erfindung, nämlich die trotz erhöhter Aussentemperatur konstante Abgabe von Wirkstoff aufgrund eines "Temperaturpuffers" im Stoffblister und die Verhinderung der Abgabe von Wirkstoff bei Überschreiten einer bestimmten Temperatur mittels eines Druckblisters, können sowohl unabhängig von einander als auch in Kombination eingesetzt werden. Die Kombination beider Vorrichtungen gestattet eine vollkommen automatische Steuerung der Abgabe der Wirkstoffe, ohne dass es dazu eines menschlichen Eingriffs bedarf.

Die weitere Aufgabe, insbesondere Krankheitserreger, unerwünschte Mikropartikel und gasförmige Substanzen aus der Luft zu entfernen, wird durch ein Kompartiment für die Stoffabsorption (Akzeptorblister, manchmal auch Akzeptorsystem genannt) innerhalb des gesamten Systems gelöst, wobei dieses Kompartiment vorzugsweise physikalisch getrennt von dem Kompartiment zur Abgabe eines Wirkstoffes (Donorblister, manchmal auch Donorsystem genannt) angeordnet ist, jedoch analog zu dem vorstehend genannten Kompartiment zur konstanten Abgabe eines Wirkstoffes aufgebaut sein kann. Der Unterschied des Absorptionssystems (auch Akzeptorsystem genannt) zum Stoffabgabesystem (Donorsystem) liegt insbesondere in der Beschichtung des Trägermaterials des Blisters. Der Stoffblister zur Entfernung von Krankheitserregern und unerwünschten Gerüchen enthält vorzugsweise ein Polyacrylharz als Bestandteil mit einem lipophilen Dispergator, der im Weiteren kationische Funktionsgruppen und/oder kationisch modifizierte Partikel enthält, sowie Enzyme.

Durch den Einsatz eines Blisters zur Abgabe eines Wirkstoffes (= Donorblister) und eines Blisters zur Absorption von unerwünschten Mikropartikeln und gasförmigen Substanzen (= Akzeptorblister) können Systeme mit synergistischen Wirkungsweisen erzeugt werden. Die Systeme - auch Hybridsysteme genannt - weisen z.B. eine Kombination von einem oder mehreren Donorblistern und einem oder mehreren Akzeptorblistem auf, die z.B. einen oder mehrere, ggf. unterschiedlich beschichtete Träger aufweisen. Dabei können die Blister parallel und/oder in Reihe angeordnet werden. Z.B. kann zunächst ein (1) Blister zur Entfernung unerwünschter Mikropartikel und gasförmiger Substanzen angeordnet werden (= Akzeptorblister), gefolgt von einem (2) Blister zur Abgabe eines Duft- oder Wirkstoffs (= Donorblister) und gegebenenfalls einem (3) Blister zur verminderten (d.h. gedrosselten oder dezenten) Stoffabgabe (= Drosselungsblister) und Erzeugung von allgemeinem Wohlbefinden, d.h. die drei Blister können in Reihe angeordnet werden, so dass die Luft der Reihe nach durch die drei Blister (1), (2) und ggf. (3) hindurchstreicht. Alternativ kann der Blister (1) parallel zu dem Blister (2) und ggf. (3) angeordnet werden, die wiederum in Reihe angeordnet sein können. Der Blister (3) zur Drosselung der Abgabe eines Wirkstoffs ist jedoch eine Option und wird lediglich bei Bedarf eingesetzt.

Das Problem der Abgabe einer bestimmten konstanten Menge an Wirkstoff, wie zum Beispiel an Duftstoff (= Riechstoff) - in verhältnismässig unabhängiger Weise von der Umgebungstemperatur - wird durch die Verwendung von bei niedriger Temperatur, bevorzugt bei 23 - 40 °C schmelzenden, tendentiell lipophilen Polymeren mit einem HLB-Wert von 8 bis 18 (Hydrophilic-Lipophilic-Balance, siehe H. Sonntag, Lehrbuch der Kolloidwissenschaft, VEB Deutscher Verlag der Wissenschaften Berlin 1977) mit einer hohen Schmelzenthalpie in der Duftstoffmischung gelöst. Durch den Einsatz derartiger Polymere mit hoher Schmelzenthalpie kommt es bei einem Anstieg der Umgebungstemperatur zu einer Verzögerung des Temperaturanstiegs in der Wirkstoffmischung, so dass die Polymere als "Temperaturpuffer" im System wirken und dadurch die Abgabe der Wirkstoffe trotz steigender Umgebungstemperatur begrenzt wird.

In derselben Richtung wirkt auch die Zugabe von wasserlöslichen organischen, meist polymeren Substanzen und/oder Elektrolyten mit endothermer Sorptions- bzw. Lösungsenthalpie in Wasser. Indem diese Substanzen bei Kontakt mit Wasser bzw. einer Erhöhung des Wasseranteils in der Blisterpackung mit einer Temperaturerniedrigung reagieren, fungieren sie ebenfalls als "Temperaturpuffer" und tragen somit ebenfalls zur Lösung des erstgenannten Problems bei, d.h. sie begrenzen die Abgabe von Wirkstoffen trotz steigender Umgebungstemperatur. Die Luft kann um so mehr Wasser als Dampf aufnehmen, je höher die Lufttemperatur ist. Bei erhöhter Lufttemperatur steigt also in der Regel der Wassergehalt der Luft an. Die vorstehend genannten Zusätze bewirken, dass bei erhöhten Temperaturen durch die Wassersorption in der Wirkstoff-Blistermasse eine Temperatursenkung erfolgt. Dieser Mechanismus funktioniert auch in umgekehrter Richtung. Da die beschriebene Temperaturpufferung die Funktion einer nur beschränkt zur Verfügung stehenden Schmelz- oder Lösungsenthalpie der Duftstoffspeichermasse ist, kann als sinnvolle Massnahme bei langfristig erhöhten oder weiter steigenden Temperaturen eine Verschliessung des Duftstoffblisters erfolgen.

Das Problem einer Verhinderung der Abgabe oder Aufnahme von erwünschten oder unerwünschten Wirksubstanzen bei Überschreiten einer bestimmten Temperatur wird gelöst, indem eine leichtflüchtige Substanz in ein geschlossenes, ausdehnungsfähiges Gefäss gefüllt wird, welches sich mit steigender Temperatur aufgrund des zunehmenden Dampfdrucks dieser Substanz ausdehnt und dadurch die Öffnung für die Abgabe der Duftstoffe bei Überschreiten einer bestimmten Temperatur verschliesst.

### Ausführungsformen der Erfindung

Die vorliegende Erfindung umfasst jede Kombination der nachfolgenden Merkmale bzw. Ausführungsformen.

Diese Erfindung betrifft ein Blistersystem mit mindestens einem ersten Blister zur Abgabe eines Wirkstoffes (= Donorblister), wobei dieser Blister c) mindestens eine Matrix, und d) mindestens einen Wirkstoff, und e) mindestens ein Polymer, dessen Schmelz- oder Zersetzungstemperatur höher als 100 °C liegt, und / oder f) mindestens ein Polymer, das bei einer Temperatur von 23 - 40 °C schmilzt umfasst, und mit mindestens einem zweiten Blister (= Akzeptorblister), wobei dieser Blister c) mindestens eine Matrix, und h) mindestens ein Kunstharz, und/oder i) mindestens einen Dispergator, und j) mindestens ein Krankheitserreger absorbierendes oder abtötendes oder hemmendes Mittel, und / oder mindestens ein Mikropartikel und/oder gasförmige Substanzen absorbierendes Mittel, und k) mindestens ein hydrolytisch wirkendes Enzym und 1) mindestens einen partikulären Träger umfasst. Das Kunstharz h) und der Dispergator i) können in dieser Zusammensetzung alternativ, d.h. jeweils einzeln oder in Kombination eingesetzt werden.

Gemäß einer bevorzugten Ausführungsform umfasst das Blistersystem eine niedermolekulare, in Wasser lösliche Substanz g) mit einer endothermen Lösungs- bzw. Sorptionsenthalpie in Wasser.

Gemäß einer weiteren bevorzugten Ausführungsform besitzt die niedermolekulare, in Wasser lösliche Substanz g) eine endotherme Sorptions- bzw. Lösungsenthalpie in Wasser von 4.0 kJ/mol - 210 kJ/mol (entsprechend ca. kcal/mol - 50 kcal/mol), vorzugsweise 40 kJ/mol - 100 kJ/mol.

Gemäß einer weiteren bevorzugten Ausführungsform enthält die niedermolekulare, in Wasser lösliche Substanz g) Harnstoff, sekundäres Natriumphosphat und/oder Kaliumnitrat.

Gemäß einer weiteren bevorzugten Ausführungsform umfaßt die Matrix c)eine poröse Matrix, wie zum Beispiel eine poröse faser- oder schaumstoffartige Matrix.

Gemäß einer weiteren bevorzugten Ausführungsform umfaßt die Matrix c) ein Faservlies aus natürlichen und/oder synthetischen Fasern, insbesondere ein Polypropylenvlies oder einen Schaumstoff, insbesondere ein Polyether-, Polyester-, Polyurethan- oder Polyamid-Schaumstoff mit offenen Poren. Wird die Matrix c) in mehreren Blistern verwendet, so kann sie jeweils gleich oder verschieden sein.

Gemäß einer weiteren bevorzugten Ausführungsform ist der Wirkstoff d) und/oder das Mittel j) verkapselt bzw. eingekapselt (= inkapsuliert), wobei das Inkapsulierungsmittel sowohl organischer als auch anorganischer Natur sein kann. Bevorzugte Materialien sind Cyclodextrin, Glucan, Chitosan und/oder Dendrimere sowie Silikatpartikel wie Zeolithe, Kaoline etc..

Gemäß einer weiteren bevorzugten Ausführungsform ist der Wirkstoff d) und/oder das Mittel j) vorzugsweise in Kombination mit Tensiden als Vesikel bzw. Liposom und/oder Mikrokapsel formuliert.

Gemäß einer weiteren bevorzugten Ausführungsform enthält das Polymer e) mindestens ein vernetzbares Polypeptid, z.B. Gelatine, Polysaccharid, z.B. Methocell, Dow Chemical Company, Hydroxyethyl Stärke, Suchema AG, Polyvinylalkohol, z.B. Mowiole Hoechst AG, oder Blockpolymerisat auf der Basis von Polyvinylalkohol und Polyvinylacetat / Polyvinylpyrrolidon, z.B. PVP / VA S-630 von ISP Global Technologies.

Gemäß einer weiteren bevorzugten Ausführungsform bildet das Polymer f) mit dem Polymeren e) und / oder dem Wirkstoff d) eine homogene Phase, eine wässrige Dispersion oder eine Emulsion.

Gemäß einer weiteren bevorzugten Ausführungsform enthält das Polymer f) ein Ethylen /Propylenoxid - Blockpolymerisat, ein Fettsäurewachs, und/oder ein Polyethylenwachs.

Gemäß einer weiteren bevorzugten Ausführungsform enthält das Polymere f) Pluronic PE 9400, und / oder Pluronic PE 10500 (BASF).

Gemäß einer weiteren bevorzugten Ausführungsform enthält das Mittel j) Silber enthaltende Mikro- und/oder Nanopartikel, polymere quaternäre Ammonium-Verbindungen, und/oder Mikro- und/oder Nanopartikel auf der Basis von Glucan, Chitosan, Microcellulose oder polymere Kieselsäure, welche mit quaternären Ammonium-Verbindungen modifiziert sind.

Gemäß einer weiteren bevorzugten Ausführungsform werden als hydrolytisch wirkende Enzyme k) Lysozym, Amylasen, insbesondere alpha-Amylase, Cellulase, Glucanase, Pektinase, und Lipase eingesetzt.

Gemäß einer weiteren bevorzugten Ausführungsform enthält der Blister mindestens ein Lösungsmittel, und/oder mindestens ein Verdickungs- und/oder Geliermittel, und/oder mindestens ein Vernetzungsmittel, und/oder mindestens ein Konservierungsmittel, und/oder mindestens einen grenzflächenaktiven Stoff.

Eine Ausführungsform der vorliegenden Erfindung betrifft insbesondere ein Blistersystem, das mindestens einen der vorstehend definierten Donorblister und mindestens einen der vorstehend definierten Akzeptorblister, und gegebenenfalls mindestens einen Druckblister umfasst, wobei der Druckblister a) ein in mindestens einer Dimension ausdehnungsfähiges Behältnis umfaßt, das b) mindestens ein niedrig siedendes Lösungsmittel enthält.

Eine weitere Ausführungsform der Erfindung betrifft ein Blistersystem mit zwei Stoffblistem (Donor- und Akzeptorblister) und mindestens einem Druckblister, wobei einer der Stoffblister zur Abgabe von Duftstoffen und/oder Arzneimitteln (Donorblister) und der andere Stoffblister zur Aufnahme von Krankheitserregern, Mikropartikeln und unerwüschten Substanzen (Akzeptorblister) dient.

Gemäß einer weiteren bevorzugten Ausführungsform enthält das Lösungsmittel b) ein Verdickungsmittel und/oder ein Sorptionsmittel.

Gemäß einer weiteren bevorzugten Ausführungsform enthält das Verdickungsmittel ein Cellulosederivat und/oder Polyvinylpyrrolidon, und/oder enthält das Sorptionsmittel chemisch modifizierte Siliciumdioxid-Nanopartikel, wie z.B. methylierte Siliziumdioxid-Nanopartikel "Aerosil R 972" der Firma Degussa.

Gemäß einer weiteren bevorzugten Ausführungsform enthält das Verdickungsmittel eine Hydroxypropyl-Methylcellulose und/oder eine Ethylhydroxyethyl-Cellulose.

Gemäß einer weiteren Ausführungsform befindet sich das Donor- und das Akzeptorsystem in einem Gehäuse.

Eine weitere Ausführungsform kann bis zu drei Stoffblister enthalten, in dem der erste die Funktion der Bakterienabsorption, der zweite die Funktion der Wirksubstanzabgabe und der dritte die Funktion zur Drosselung der Wirksubstanzabgabe besitzt.

### Detaillierte Beschreibung

In der gesamten Anmeldung sind % Gewichtsprozente und Teile Gewichtsteile, sofern nicht anders angegeben. Unter dem Begriff "Blister" wird im Allgemeinen ein Behälter, eine Packung, ein Gefäß oder eine Vorrichtung verstanden, die bzw. der eine bestimmte Mischung enthält, deren Zusammensetzung von dem beabsichtigten Verwendungszweck abhängt. Unter dem Begriff "Mikropartikel" werden Teilchen mit einer Teilchengröße im Bereich von 1 µm bis 1000 µm, insbesondere von 1 µm bis 10 µm verstanden; Unter dem Begriff "Nanopartikel" werden Teilchen mit einer Teilchengröße im Bereich von 1 nm bis 1.000 nm, insbesondere von 1 nm bis 500 nm verstanden.

### Verschlussmechanismus mittels Druckblister

Das Blistersystem kann ggf. einen sich eigendynamisch regulierenden physikalisch/chemischen Verschlussmechanismus durch einen Druckblister (= Druckbehälter) enthalten. Der vorzugsweise luftdicht verschlossene Druckblister kann als eine Möglichkeit verwendet werden, den Stoffblister (insbesondere den Donorblister) temperaturabhängig zu verschließen. Der Druckblister enthält mindestens ein Lösungsmittel bzw. Lösungsmittelgel b), dessen Dampfdruck in dem Druckblister einen temperaturabhängigen Druck erzeugt. Der bei Temperaturerhöhung innerhalb des Druckblisters zunehmende Druck wird zur Auslösung des Verschlussmechanismus' benutzt, so dass die weitere Zufuhr von Duftstoffen unterbunden wird. Der Druckblister stellt eine Option dar und wird bevorzugt dann eingesetzt, wenn es gilt, eine übermäßige Abgabe des Wirkstoffes an die Raumluft, insbesondere bei erhöhten Temperaturen, vorzugsweise bei mehr als 35 °C, zu verhindern.

Die chemische Zusammensetzung der in dem Druckblister einzusetzenden Gelmasse, d.h. das verdickte bzw. sorbierte Lösungsmittel b), orientiert sich an der zur Schliessung des Systems notwendigen Kraft und somit an der individuellen Konstruktion des Wirkstoffdispensers.

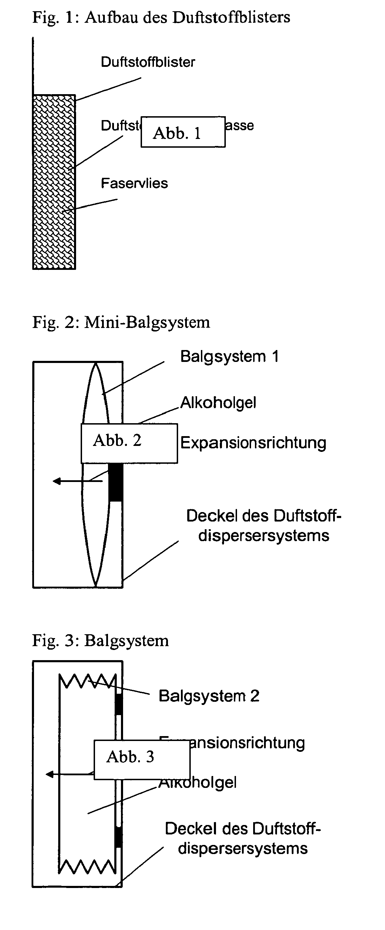

Die Konstruktion des **Behältnisses a)** des Druckblisters hängt ebenfalls vornehmlich vom Verwendungszweck ab, und kann so konzipiert sein, dass die druckbedingte Ausdehnung des Behältnisses a) nur eindimensional ist. Es sind aber auch Ausdehnungen in zwei oder drei Dimensionen möglich. Als Beispiele für das Behältnis a) kommen z.B. ein Minibalgsystem (Fig. 3) oder ein rotationssymetrischer, sich nur eindimensional ausdehnender Ballon (Fig. 2) in Betracht. Die Ausdehnungsdistanzen des Druckblisters in der gewünschten, zum Verschliessen des Wirkstoffblisters notwendigen Richtung betragen z.B. 1 - 20 mm, vorzugsweise 5 - 10 mm. Der Druckblister kann u.a. aus elastischen, gegenüber den verwendeten Lösungsmitteln beständigen Kunststoffen aufgebaut sein, die gegebenenfalls z.B. durch eine Aluminiumschicht für Lösungsmitteldampf undurchlässig gemacht sind.

Das Kernstück des Druckblisters ist mindestens ein in mindestens einer Dimension ausdehnungsfähiges Behältnis, in dem sich ein niedrig siedendes, vorzugsweise bei 40 - 100 °C, bevorzugt bei 50 -90 °C, besonders bevorzugt bei 60 bis 80 °C siedendes **Lösungsmittel bzw. Lösungsmittelgemisch b)** befindet. Steigt infolge einer höheren Umgebungstemperatur der Dampfdruck des Lösungsmittels im Druckgefäss an, so dehnt sich dieses aus und verschliesst die Öffnung für die Abgabe der Wirkstoffe mechanisch; die Abgabe der Wirkstoffe wird somit mit dem Überschreiten einer bestimmten Temperatur unterbunden, die von der Zusammensetzung des Gemisches im Druckgefäss abhängt.

Zur Regulierung des von der Umgebungstemperatur abhängigen Dampfdrucks werden vorzugsweise Gemische von einzelnen Lösungsmitteln b) eingesetzt, die sich durch verschiedene Siedepunkte und/oder Verdampfungsenthalpien unterscheiden. Weitere den Dampfdruck bestimmende mögliche Zusätze sind der Polarität der Lösungsmittel angepasste Polymere, z.B. Poly(Methyl vinyl ether / Maleinsäure) "Gantrez SP-215", oder Lösungsmittel sorbierende Partikel, z.B. methylierte Silizium-Nanopartikel "Aerosil R 972" der Firma Degussa, die in Kombination mit dem Lösungsmittel Gele ausbilden.

Als geeignete Lösungsmittel für den Druckblister kommen bspw. Wasser, Methanol, Ethanol, Isopropanol, und Aceton in Betracht; insbesondere werden Alkohol/Wassermischungen eingesetzt.

Als Beispiele für Verdickungsmittel für den Druckblister kommen Cellulosederivate und Polyvinylpyrrolidon in Betracht; insbesondere werden Hydroxypropyl-Methylcellulose (entspricht Methocell von Dow-Chemical Company), Ethylhydroxyethyl-Cellulose (entspricht Bermocoll E 230 FQ von AKZO Nobel) oder Polyvinylpyrrolidon, z.B. PVP K-120 der Firma ISP, als Verdickungsmittel eingesetzt. Die hierfür eingesetzten Polymerkonzentrationen betragen z.B. 0.5 - 10 %, vorzugsweise 1 - 5 %, bezogen auf die gesamte Gelmasse.

Als typische Lösungsmittel sorbierende anorganische Substanzen sind z.B. unterschiedlich modifizierte Siliziumdioxid-Nanopartikel der Firma Degussa AG, z.B. methylierte Silizium-Nanopartikel "Aerosil R 972" der Firma Degussa, zu erwähnen.

### Stoffblister zur Abgabe eines Wirkstoffes (auch Donorblister genannt)

Der Stoffspeicher, auch als Stoffblister, Stoffbehälter oder als Donorblister bezeichnet, umfasst mehrere Komponenten, die ihrerseits Träger der zur Aufgabenerfüllung notwendigen Funktionen sind. Unter einem Donorblister wird ein Behälter verstanden, der für die Aufnahme der Bestandteile c), d), e), f) und g) vorgesehen bzw. geeignet ist.

Als eine erste Komponente ist mindestens eine **Matrix c)** als mechanisches "Gerüst" des Stoffspeichers zu nennen; die Matrix ist vorzugsweise porös; besonders bevorzugt ist die Matrix so porös, dass sie die Komponenten d), e), f), und g) vollständig oder zumindest zu 90% bzw. zu 80% aufnehmen kann. Diese Matrix ist z.B. ein - insbesondere poröses - faser- oder schaumstoffartiges Flächengebilde bzw. Matrix. Vorzugsweise werden Faservliese mit einer Dicke von 1 bis 10 mm aus nativen und/oder Synthesefasern verwendet, die mit der Duftstoffträgermasse bzw dem Arzneimittel z.B. besprüht, getränkt oder beschichtet werden (siehe Fig. 1). Besonders bevorzugt ist eine poröse Matrix aus Polypropylenvlies, z.B. von der Firma Freudenberg AG, oder von der Firma Sandler AG. Es ist auch möglich, mehrere, auch beschichtete Vlieslagen als gestapeltes Paket zu einem Duftstoffblister zu konfektionieren. Ebenso kann ein Schaumstoff, vorzugsweise ein Schaumstoff mit offenen Poren, verwendet werden, insbesondere ein Polyether-, Polyester-, Polyurethan- oder Polyamid-Schaumstoff mit offenen Poren.

Die zweite Komponente für den Stoffblister umfasst einen Duftstoff oder eine **Duftstoffmischung d)**. Bezüglich der Wahl der Duftstoffe ist das beschriebene System völlig offen, so dass allein der Anwendungsbereich des Duftstoffdispensers für die Auswahl der Duftsubstanz bestimmend ist. Insbesondere werden Duftstoffe aus der Terpenklasse verwendet, wie zum Beispiel Citronellol, Citronellal, Hydroxycitronellol, Hydroxycitronellal, Nerolidol, Linalool.

Der Stoffblister kann auch ein oder mehrere Arzneimittel enthalten, das/die entweder allein oder in Verbindung mit einem oder mehreren Duftstoffen als "Wirkstoff" eingesetzt werden kann/können. Bevorzugt werden Arzneimittel mit beruhigender Wirkung eingesetzt, wobei der Bedarf solcher Stoffdispensersysteme speziell im Krankenhausbereich liegt. Als Beispiel für ein Arzneimttel kann Happybelle PE dienen, ein Phytoendorphin Extrakt der Fa. Mibelle AG (Buchs, Schweiz), welcher analgetisch und euphorisierend wirkt, und somit zum allgemeinen Wohlbefinden beiträgt. Weiter für diesen Bereich einsetzbare Substanzen sind: Mandarinen-, Orangen- und Melissenöl sowie Hopfenöl und Passionsblumenextrakt, wobei letztere zwei sedierende Wirkung besitzen.

Duftstoffe und/oder Arzneimittel werden im Sinne der vorliegenden Erfindung als Wirkstoffe bezeichnet.

Durch die Inkapsulierung der Duftstoffe bzw. Arzneimittel kann deren Abgabegeschwindigkeit verzögert bzw. vergleichmässigt werden (z.B. Stoffabgabe gemäss einer Reaktion nullter Ordnung). Zusätzlich können dadurch auch weitere, unterschiedliche Funktionalitäten, z.B. allgemeines Wohlbefinden durch Happybelle PE der Fa. Mibelle AG, integriert werden. Zur Inkapsulierung der Duftstoffe bzw. Arzneimittel können organische Materialien, wie zum Beispiel Cyclodextrine, Glukan, Chitosan und/oder Dendrimere verwendet werden. Ebenfalls geeignet sind anorganische Materialien, wie Silikatpartikel, insbesondere Zeolithe, Kaoline, etc.. Ebenso können die Duftstoffe bzw. Arzneimittel in Kombination mit Tensiden als Vesikeln, Liposomen, Nano- oder Mikropartikel formuliert werden.

Eine weitere Möglichkeit, eine Stoffabgabe von näherungsweise nullter Ordnung zu erzielen, ist die Emulgierung der Duftstoffe bzw. Arzneimittel in mindestens ein geschmolzenes Polymer f) (siehe unten) mittels grenzflächenaktiver Stoffe (= Tenside).

Durch die langsame Abkühlung der so entstandenen Emulsion verfestigt sich diese unter Ausbildung von nanoskopischen "Containern", in denen der Duftstoff oder die Substanzen mit einer anderen Funktionalität eingeschlossen sind, z.B. Beruhigungsmittel. Als optional einsetzbare grenzflächenaktive Stoffe (= Tenside) zur Emulgierung werden z.B. ethoxylierte Fettalkohole, Glycerinester oder sulfonierte Lauryl- oder Stearylderivate eingesetzt. Der HLB-Wert (= Hydrophilic-Lipophilic Balance) dieser Substanzen bewegt sich im Bereich von 8 - 18, bevorzugt 9 - 16, insbesondere bevorzugt 10 - 14.

Eine weitere Komponente des Stoffblisters ist ein **Polymer e),** dessen Schmelz- bzw. Zersetzungstemperatur über 100°C liegt. Die Funktion dieses Bestandteils des Stoffblisters betrifft die chemische Stabilisierung der Duftstoff- bzw. Arzneimittel-Trägermasse, um bei erhöhten Temperaturen ein Fliessen der Trägermasse zu verhindern.

Als geeignete Substanzen für das **Polymer e)** werden vorzugsweise Substanzen eingesetzt, die nicht unter 100 °C schmelzen bzw., die sich erst bei einer Temperatur von oberhalb von 100°C zersetzen. Hierfür geeignet sind beispielsweise chemisch vernetzbare Polypeptide, zum Beispiel Gelatine; Polysaccharide, z.B. Methocell, Dow Chemical Company, Hydroxyethyl Stärke, Suchema AG; Polyvinylalkohole, z.B. Mowiole Hoechst AG; oder Blockpolymerisate auf der Basis von Polyvinylalkohol und Polyvinylacetat / Polyvinylpyrrolidon, z.B. PVP / VA S-630 von ISP Global Technologies. Ebenfalls geeignet sind sind Fettkohlenwasserstoff-Derivate, wie zum Beispiel C₁₂-C₂₄-Fettsäuren und deren Ester. Die Einsatzmenge des Polymers e) liegt je nach verwendetem Polymer f) bei 20 - 150 g/l des wässrigen Gels, bezogen auf die Gelmasse des Stoffblisters.

Eine weitere Komponente für den Stoffblister betrifft das **Polymer f),** welches vorzugsweise bei einer niedrigen Temperatur, insbesondere bei 23 bis 40°C, insbesondere bevorzugt bei 25 - 30°C schmilzt. Insbesondere besitzt dieses Polymer f) eine hohe Schmelzenthalpie. Als Schmelzenthalpie wird die bei der Phasenumwandlung vom festen in den flüssigen Aggregatzustand umgesetzte so genannte "latente" Wärme bezeichnet. Die Schmelzenthalpie der hierzu benutzten Polymere liegt z.B. im Bereich von 50 - 500 J/g Polymer, vorzugsweise im Bereich von 150 - 250 J/g Polymer. Bei monomolekularen Stoffen werden die thermodynamischen Daten wie Schmelzenthalpie oder Lösungs- bzw. Sorptionsenthalpie in cal/mol bzw. J/mol angegeben, bei polymeren Stoffen dagegen in cal/g Polymer bzw. J/g Polymer.

Das **Polymer f)** dieser Erfindung kann der Duftstoffaufnahme und/oder Arzneimittelaufnahme, Speicherung und "Wärmeregelung" - im Sinne eines "Temperaturpuffers" - des Stoffblisters dienen. Es umfasst bei niederer Temperatur schmelzende, tendenziell lipophile Polymere mit einer möglichst hohen Schmelzenthalpie. Dies hat zur Folge, dass trotz erhöhter Umgebungstemperatur die Temperatur des Stoffblisters während einer längeren Zeit nicht über die Schmelztemperatur der zur Temperaturkontrolle eingesetzten Polymere ansteigt, da aufgrund der hohen Schmelzenthalpie eine grosse Wärmemenge für den Phasenübergang von fest nach flüssig erforderlich ist. Dadurch kann der Temperaturanstieg im Stoffblister aufgrund einer hohen Umgebungstemperatur gebremst werden.

Das Polymer f) ist ein bei niederen Temperaturen, vorzugsweise bei 23 - 40 °C, bevorzugt bei 25 - 30°C schmelzbares Polymer, welches aufgrund seiner chemischen Konstitution sowohl in wässrigen als auch in anderen polaren Lösungsmitteln löslich ist und mit dem Polymer e) und / oder dem Duftstoff bzw. Arzneimittel d) und ggf. der niedermolekularen, wasserlöslichen Substanz g) entweder eine homogene Phase oder eine wässrige Dispersion bzw. Emulsion ausbildet.

Bevorzugt als Polymer f) eingesetzte Substanzen sind z.B. Ethylen/Propylenoxidblockpolymerisate oder Fettsäure- und Polyethylenwachse. Bevorzugt werden Pluronic PE 9400 und Pluronic PE 10500 (beides sind Markennamen der BASF und bezeichnen Ethylenoxid-Propylenoxid-Polymere) als Polymer f) verwendet.

Die Temperaturpufferkapazität wird einerseits durch die chemische Natur der Polymersubstanzen und deren Schmelzenthalpie, andererseits durch den eingesetzten Anteil des Polymers bestimmt. Die zu diesem Zweck eingesetzten Polymerkonzentrationen liegen z.B. bei 30 - 70 Gew.-%, bezogen auf die gesamte Duftstoffspeichermasse.

Aufgrund des breiten Löslichkeitsspektrums der in dieser Komponente zusammengeführten Ingredienzien sind die insbesondere lipophilen Duftstoffe bzw. Arzneimittel direkt oder in Kombination mit grenzflächenaktiven Stoffen mischbar. Als grenzflächenaktive Stoffe werden z.B. ethoxylierte Fettalkohole, Glycerinester oder sulfonierte Lauryl- oder Stearylderivate eingesetzt. Der HLB-Wert (Hydrophilic-Lipophilic Balance) dieser Substanzen bewegt sich im Bereich von 8 - 18, bevorzugt von 9-16, insbesondere bevorzugt von 10-14.

Ein weiterer Bestandteil der Mischung für den Stoffblister, der vorzugsweise enthalten sein kann, umfasst eine **niedermolekulare, wasserlösliche Substanz g) mit einer endothermen Lösungs- bzw. Sorptionsenthalpie** in Wasser. Als Lösungsenthalpie wird jene Wärmemenge [kcal/mol] bezeichnet, die z.B. beim Lösen einer Substanz in Wasser frei wird (exotherm) oder aufzuwenden (endotherm) ist. Als Sorptionsenthalpie eines Stoffes bezüglich Wasser bezeichnet man jene Wärmemenge [kcal/mol bzw. kJ/mol], die entweder freigesetzt wird (= exotherm) oder aufgewendet werden muß (= endotherm), wenn Wasser bzw. Wasserdampf von diesem Stoff, der mit dem Wasserdampf in Berührung steht, aufgenommen wird (Römpp: Chemie Lexikon, Band 5, Thieme, Stuttgart 1992).

Die niedermolekulare, wasserlösliche Substanz g) mit einer endothermen Lösungs- bzw. Sorptionsenthalpie in Wasser dient dazu, das Wasser der Umgebungsluft zu sorbieren und durch die endotherme Sorptionsenthalpie die Temperatur des Stoffblisters mit der Trägermasse zu senken. Solche Substanzen mit endothermer Lösungs- bzw. Sorptionsenthalpie wirken in der vorliegenden Mischung für den Stoffblister als "Temperaturpuffer" in dem Sinne, dass sie einen Anstieg der Temperatur der Masse in dem Stoffblister verhindern bzw. die Temperatur der Masse in dem Stoffblister herabsetzen, wenn die (absolute) Luftfeuchtigkeit der Umgebung, und damit in der Regel auch die Temperatur der Umgebung zunimmt.

Die hohe Schmelzenthalpie des Polymers f) sowie die endotherme Sorptions- bzw. Lösungsenthalpie in Wasser der niedermolekularen, wasserlöslichen Substanz g) der Stoffzusammensetzung für den Stoffblister sind beide für den "Temperaturpuffer" verantwortlich, aufgrund dessen die von der Umgebungstemperatur relativ unabhängige, gleichmässige Abgabe der Duftstoffe aus der Mischung erfolgt. Sie wirken insofern in die gleiche Richtung, d.h. synergistisch.

Beispiele für eine niedermolekulare, wasserlösliche Substanz g) mit einer endothermen Lösungs- bzw. Sorptionsenthalpie in Wasser sind Harnstoff mit einer Lösungsenthalpie von 15,5 kJ/mol (bzw. 3,7 kcal/mol) sowie dessen Derivate, aber auch anorganische Salze wie z.B. Dinatriumhydrogenphosphat (Na₂HPO₄ · 12 H₂O) mit einer Lösungsenthalpie von 93,9 kJ/mol (bzw. 22,4 kcal/mol), oder Kaliumnitrat (KNO₃). Die Einsatzmengen typischer, Wasser-sorbierender Mittel mit endothermer Lösungswärme liegen bei 0,1 - 10 Gew.-%, bevorzugt bei 0,2 - 5,0 Gew.-%, besonders bevorzugt bei 0,2 - 2,0 Gew.-%, insbesondere bevorzugt bei 0,2 - 1,0 Gew.-%, bezogen auf die gesamte Duftstoffspeichermasse.

Die temperaturunabhängige, gleichmässige Duftstoff- bzw. Arzneimittel-Abgabe, basierend auf dem beschriebenen Funktionsprinzip erlaubt es, auf der Stoffblisterhülle eine für den Benutzer sichtbare Markierung anzubringen, die ein zur noch im Stoffblister vorhandenen Duftstoff- bzw. Arzneimittelmenge proportionales Signal abgibt, um so dem Benutzer den Zeitpunkt des Blisteraustausches zu markieren. Möglichkeiten hierzu bestehen z.B. in der Applikation beschränkt lichtechter oder sauerstoffunbeständiger Farbstoffe, deren Ausbleichungszeit mit der Duftstoffspeicherkapazität korreliert.

Darüber hinaus kann der Stoffblister mindestens ein Lösungsmittel enthalten. Beispiele hierfür sind Wasser, Methanol, Ethanol, Isopropanol und Aceton, insbesondere Wasser und Methanol, sowie deren Gemische.

Darüber hinaus kann der Stoffblister mindestens ein Verdickungs- und / oder Gelierungsmittel enthalten. Beispiele hierfür sind Gelatine, Cellulose- und Guar-Derivate, Alginate und Polyvinylpyrrolidon. Als Beispiel können in hydrophober Weise modifizierte, ethoxylierte Urethane genannt werden, die zur Selbstassoziation neigen und zusammen mit grenzflächenaktiven Mitteln die Viskosität beeinflussen. Ein konkretes Beispiel eines Verdickungsmittels ist Borchi Gel L75N.

Darüber hinaus kann der Stoffblister mindestens ein Vernetzungsmittel enthalten. Beispiele hierfür sind Glutaraldehyd, Formaldehyd, Trioxan, methoxylierte Ethylenharnstoff- und Melaminderivate.

Darüber hinaus kann der Stoffblister mindestens ein Konservierungsmittel enthalten. Beispiele hierfür sind Harnstoff - Peroxid - Additionsprodukt, Imidazolidinylharnstoff, Diazolidinylharnstoff, Methylparaben, Propylparaben.

Darüber hinaus kann der Stoffblister mindestens einen grenzflächenaktiven Stoff enthalten. Beispiele hierfür sind ethoxylierte Fettalkohole, Glycerinester oder sulfonierte Lauryl- oder Stearylderivate. Der HLB-Wert (Hydrophilic-Lipophilic Balance) dieser Substanzen bewegt sich vorzugsweise im Bereich von 8 -18, bevorzugt von 9 - 16, insbesondere bevorzugt von 10 - 14.

Die Herstellung des Stoffblisters geschieht zum Beispiel durch das Zusammenfügen bzw. Einmischen der Komponenten d), e), f) und ggf. g) zu einer Masse, die auf die vorzugsweise poröse Matrix c) appliziert wird. (siehe Beispiele). Anschließend wird bei Anwesenheit von Lösungsmitteln nach dem Auftragen auf die Matrix c) ein Trocknungsprozess nachgeschaltet, so dass zumindest ein Teil des Lösungsmittels verdunstet.

Die gegenseitige mechanische Unabhängigkeit der Stoffblister und ggf. des Druckblisters machen es möglich, dass sowohl das gesamte Stoffdispensersystem als Wegwerfartikel als auch als nachfüllbares System mit den jeweils einzelnen Systemkomponenten (Stoff-, d.h. Donor- oder Akzeptorblister, bzw. Druckblister) gefertigt werden kann. Diese Möglichkeit birgt den Vorteil, Designer-Systeme herzustellen, deren Stoffblister durch andere Stoffblister z.B. mit zusätzlichen Funktionalitäten austauschbar sind.

Als Gehäuse für Druckblister, Donorblister und/oder Absorberblister, bzw. für das Stoffdispensersystem insgesamt, ist jegliches Behältnis geeignet, das zumindest einen Druckblister und zumindest einen Stoffblister aufnehmen kann. Beispiele derartiger verschliessbarer Behältnisse sind Spritzgussboxen oder auch extrudierte oder auf andere Weise geformte Kunststoffboxen. Das Gehäuse für das Stoffdispensersystem umfaßt einen wärmeisolierenden oder geschäumten Kunststoff, oder eine doppelwandige Konstruktion, in deren Zwischenraum sich eine wärmeisolierende, und/oder schmelzbare Wachsmasse befindet. Als Materialien für das Gehäuse kommen Kunststoff, metallische oder cellulosische Werkstoffe, oder Verbundwerkstoffe der genannten Materialien in Betracht.

### Stoffblister zur Entfernung von Krankheitserregern, unerwünschten Mikropartikeln und gasförmigen Substanzen (auch Akzeptorblister genannt)

Der Blister kann z.B. Komponenten einer ähnlichen Zusammensetzung wie der Stoffblister zur Abgabe eines Wirkstoffes (Donorblister) aufweisen. So kann zum Beispiel die gleiche Matrix c) als Träger verwendet werden; im Allgemeinen kann jedoch die Matrix c) des Donorblister von der Matrix c) des Akzeptorblisters verschieden sein. Daher wird bezüglich der Komponenten des Akzeptorblisters, soweit sie erforderlich sind, vorzugsweise auf die Komponenten c) bis g) des Stoffblisters zur Abgabe eines Wirkstoffes (Donorblisters) verwiesen.

Das Wirkprinzip des Akzeptorblisters beruht z.B. darauf, dass die Krankheitserreger bzw. die die Luft belastenden Mikropartikel, wie Staubteilchen, Feinstaub, Zigarettenrauch und dergleichen, sowie gasförmige Substanzen, wie Schweiß, von den partikulären Trägern absorbiert bzw. adsorbiert, d.h. immobilisiert und somit aus der Luft entfernt werden. Anschließend werden die Zellwände der auf den partikulären Trägern adsorbierten Mikroorganismen durch die in der Mischung enthalten Enzyme abgebaut und die Mikroorganismen somit insgesamt unschädlich gemacht.

Darüber hinaus kann der Blister zur Entfernung von Krankheitserregern, unerwünschten Mikropartikeln und gasförmigen Substanzen (= Akzeptorblister) ein **Kunstharz h)** als Bestandteil enthalten. Als Kunstharz kommt z.B. ein Polyacrylatharz, Polyamid, Polyurethan, Cellulose-Derivat oder Copolymer oder Mischungen der genannten Polymere in Betracht. Diese können sowohl hydrophilen als auch lipophilen Charakter haben.

Diese Stoffe dienen als Bestandteil für die Beschichtungsmassen des Akzeptorblisters zur Absorption von Krankheitserregern und Mikropartikeln. Besonders geeignet sind Polyacrylate, ein Beispiel hierfür ist Carbopol EZ-2. Weitere Acrylharz basierende, die Rheologie bestimmende Verbindungen sind Carbopol ETD 2691 (Noveon), Acrysol ASE-60 (Röhm und Haas) und Acrysol TT-615 (Rohm and Haas). Die eingesetzten Mengen betragen, 0,5 bis 70 Gew.-%, vorzugsweise 1 bis 30 Gew.-%, bezogen auf die gesamte Trockenmasse der Blistermischung, d.h. ohne Berücksichtigung von Wasser.

Darüber hinaus kann der Akzeptorblister einen vorzugsweise lipophilen **Dispergator i)** enthalten. Vorzugsweise werden amphiphile Carboxylgruppen enthaltende Polymere eingesetzt, die vorzugsweise Copolymere aus ethoxyliertem Maleat und 1-Dodecen darstellen. Ein Beispiel hierfür ist Dapral GE 202. Weitere auf Acrylatbasis eingesetzte Emulgatoren sind Pemulen 1621 und Pemulen 1622.Die eingesetzten Mengen betragen, 0,5 bis 20 Gew.-%, vorzugsweise 3 bis 8 Gew.-%, bezogen auf die gesamte Trockenmasse der Blistermischung, d.h. ohne Berücksichtigung von Wasser.

Die Komponenten h) und i), d.h. das Kunstharz und der Dispergator, können alternativ eingesetzt werden, d.h., sie können jeweils einzeln oder in Kombination eingesetzt werden. Das Kunstharz h) kann durch den Dispergator i) zumindest teilweise substituiert werden, und vice versa. Der Grund hierfür liegt darin, dass diese Dispergatoren vernetzbar sind, so dass sie sich wie ein Kunstharz verhalten. Carboxylgruppenhaltige Verbindungen (z.B. Acrylharze, Dapral GE 202) werden mit Aciridinen vernetzt, während hydroxylgruppenhaltige Verbindungen wie z.B. Cellulose- und Stärkederivate, oder Sorbitanester mit Isocyanaten oder Formaldehyd-Hamstoff-Derivaten vernetzt werden.

Ferner kann der Akzeptorblister eine **bakterizid oder bakteriostatisch wirkende Substanz j)** enthalten. Als Beispiele hierfür können genannt werden: Silber enthaltende Mikro- und Nanopartikel, polymere quaternäre Ammonium-Verbindungen, sowie Mikro- und Nanopartikel auf der Basis von Glucan, Microcellulose oder polymere Kieselsäure, welche mit quaternären Ammonium-Verbindungen modifiziert wurden.

Insbesondere wird eine bakterien-immobilisierende, bakterizide/bakteriostatische Funktion dadurch erhalten, dass organische Polymere, insbesondere auf der Basis von Guar, modifizierte Cellulose, Polyamid oder Polyvinylalkohol, vorzugsweise solche mit nukleophilen Gruppen, mittels quaternärer Ammoniumverbindungen derivatisiert werden. Es können auch die unter Punkt h) genannten Kunstharze als Ausgangsmaterialien für die Derivatisierung verwendet werden. Als Beispiele für die Derivatisierungsmittel können erwähnt werden: 3-Chlor-2-hydroxypropyl-trimethylammoniumchlorid, 2,3-Epoxypropyltrimethylammoniumchlorid, 3-Chlor-2-hydroxypropyl-lauryl-dimethylammonium chlorid, 3-Chlor-2-hydroxypropyl-stearyl-dimethylammoniumchlorid, 3-Chlor-2-hydroxypropyl-benzyl-dimethylammoniumchlorid, 2,3-Epoxypropyl-benzyl-dimethylammoniumchlorid. Die kationischen Funktionsgruppen können sowohl auf dem Kunstharz als auch auf dem lipophilen Dispergator angeordnet werden. Der Anteil der Bakterien-immobilisierenden, bakteriziden/bakteriostatischen Substanz j) beträgt 1 - 20 %, vorzugsweise 4 - 8 %, bezogen auf die gesamte Trockenmasse, d.h. ohne Berücksichtigung von Wasser.

Als Beispiel für ein silberhaltiges bakterienhemmendes System kann erwähnt werden: Nano-Silber-Systeme wie z. B. Cocair der Firma ChemBotec/D. Die Einsatzmengen betragen 0.2 - 10 % vorzugsweise 1 - 3 %, bezogen auf die gesamte Trockenmasse, d.h. ohne Berücksichtigung von Wasser.

Ferner kann der Akzeptorblister ein **Enzym k)** enthalten, das die Zellwände der adsorbierten Mikroorganismen hydrolysiert. Der Sinn der Zerstörung der Zellwand der adsorbierten Bakterien besteht darin, deren möglichst rasche Abtötung herbeizuführen. Als Enzyme kommen insbesondere Hydrolasen in Betracht, vorzugsweise Lysozym zum Abbau von Peptidoglycan, alpha-Amylase, Cellulase, Glucanase, Pektinase, und Lipase. Der Enzymanteil in der gesamten Trockenmasse der Blistermischung beträgt 0,5 bis 10 Gew.-%, vorzugsweise 1 bis 3 Gew.-%.

Ferner kann der Akzeptorblister einen **partikulären Träger l)** enthalten, der für die Adsorption der Krankheitserreger und der die Luft belastenden Mikropartikel vorgesehen ist. Der Unterscheid zwischen der Bakterien-immobilisierenden, bakteriziden/bakteriostatischen Substanz j) und dem partikluären Träger 1) liegt darin, dass die Substanz j) keinerlei Quartär-Struktur aufweist, während der partikuläre Träger 1) eine Quartär-Struktur mit großen inneren Oberflächen und Hohlräumen aufweist, welche dem Grunde nach mit der Struktur von Aktivkohle, Zeolithen oder Katalysator-Trägermaterialien vergleichbar ist. So weisen zum Beispiel die inneren Hohlräume der partikulären Träger 1) Abmessungen von von 2 bis 400 nm auf.

Die Funktion der partikulären Träger 1) besteht einerseits darin, Mikroorganismen zu adsorbieren, und andererseits darin, Mikro- und Nano-Partikel, wie z.B. Feinstaub, in den Poren und Hohlräumen des Partikels zu adsorbieren und einzulagern. Das hat zur Folge, dass sich Mikroorganismen, wie zum Staphylococcus, der eine Abmessung von ca. 2 bis 3 µm aufweist, nur in geringem Maße in die Hohlräume der partikulären Träger einlagern können, während Feinstaub ohne Weiteres eindringen und adsorbiert werden kann.

Beispiele quaternär modifizierter partikulärer Träger sind: Glucan, mikronisierte Cellulose, Chitosan, und Polyamid. Zur Modifikation der organischen Partikel werden vorzugsweise quaternäre Ammoniumverbindungen eingesetzt, die eine Epoxid- oder eine Chlorhydrin-Gruppe aufweisen, insbesondere 3-Chlor-2-hydroxypropyltrimethylammoniumchlorid, 2,3-Epoxypropyltrimethylammoniumchlorid, 3-Chloro-2-hydroxypropyl-lauryl-dimethylammoniumchlorid, 3-Chlor-2-hydroxypropyl-stearyl-dimethylammoniumchlorid, 3-Chlor-2-hydroxypropyl-benzyl-dimethylammoniumchlorid, 2,3-Epoxypropyl-benzyl-dimethylammoniumchlorid eingesetzt.

Als Beispiel eines anorganischen partikulären Trägers ist polymere Kieselsäure zu erwähnen, die mit quaternären Ammoniumverbindungen, die eine hydrolysierbare Silylgruppe aufweisen, derivatisiert wird. Beispielsweise kann die Derivatisierung mit folgenden Reagenzien erfolgen: Benzyldimethyl-trialkoxysilyl-alkyl-ammoniumchlorid, wobei die Alkylgruppe 2 bis 20 Kohlenstoffatome aufweist, wie z.B. Benzyldimethyl-(trimethoxysilylpropyl)-ammoniumchlorid oder Benzyldimethyl(triethoxysilylpropyl)-ammoniumchlorid. Ebenso können verwendet werden: Alkyldimethyl-trialkoxysilylpropyl-ammoniumchloride, wobei die Alkylgruppe 2 bis 20 Kohlenstoffatome aufweist, wie z.B. Hexadecyl-dimethyl-(trimethoxysilylpropyl)-ammoniumchlorid, Octadecyl-dimethyl-(triethoxysilylpropyl)-ammoniumchlorid. Der Anteil der partikulären Träger 1) in der gesamten Trockenmasse der Blistermischung beträgt 1 bis 20 Gew.-%, vorzugsweise 4 bis 8 Gew.-%.

### Drosselungsblister

Es kann auch ein Stoffdispensersystem mit zwei Stoffblistern (Donor- und Akzeptorblister) und ggf. mindestens einem Drosselungsblister verwendet werden, welcher vorzugsweise nicht beladen ist. Durch die Verwendung nicht mit Wirkstoff beladener Blister können sowohl Wirkstoffe als auch unerwünschte Substanzen absorbiert werden. Der Drosselungsblister enthält vorzugsweise keinen Wirkstoff, bzw. er wird bei der Herstellung nicht mit Wirkstoff beladen. Er enthält vorzugsweise Aktivkohle oder Mischungen, wie sie für den Donorblister verwendet werden, jedoch ohne Wirkstoff.

Wird der nicht mit einem Wirkstoff beladene Blister (= Drosselungsblister) als erster, und die beladenen Stoffblister (Donor- bzw. Akzeptorblister) als zweiter bzw. dritter bezüglich der Luftströmung angeordnet, so wird zunächst der unerwünschte Stoff der Umgebungsluft im ersten Blister absorbiert, und die Luft wird durch den zweiten bzw. dritten beladenen Stoffblister mit dem Wirkstoff angereichert. Schließlich kann bei Bedarf ein dritter Blister zur Drosselung der Abgabe des Wirkstoffes in Reihe angeordnet werden.

Eine bevorzugte Anordnung ist: (1) Blister zur Absoprtion von Mikroorganismen und die Luft belastenden Mikropartikeln (Akzeptorblister), (2) Blister zur Abgabe eines Wirkstoffes (Donorblister), (3) Drosselungsblister, wobei alle drei Blister der Reihe nach von der Luft durchströmt werden. Eine bevorzugte Alternative ist ferner: Der Blister zur Absoprtion von Mikroorganismen und die Luft belastenden Mikropartikeln (Akzeptorblister) (1) und der Blister zur Abgabe eines Wirkstoffes (Donorblister) (2) werden parallel angeordnet; anschließend, d.h. in Strömungsrichtung der Luft, befindet sich der Drosselblister (3) in Reihenschaltung zu den beiden Blistern (1) und (2).

Ist jedoch der mit Wirkstoff beladene Blister (Donorblister) vorgeschaltet, d.h. als in Strömungsrichtung der Luft als erster angeordnet, und der nicht beladene Stoffblister nachgeschaltet (als zweiter in Strömungsrichtung der Luft angeordnet), so wird der aus der vorgeschalteten Einheit abgegebene Wirksoff von der nachgeschalteten teilweise absorbiert, wodurch gesamthaft eine abgeschwächte Wirkstoffabgabe an die Umluft resultiert. Dies funktioniert jedoch nur solange, bis in den beiden Blistereinheiten (Donor- und Drosselungsblister) dieselbe (abgesenkte) Wirkstoff-Konzentration vorherrschend ist. Nach Erreichen dieses Gleichgewichts fungiert die vormals als Drosselungsblister eingesetzte Einheit als Donor-Blister, wobei nun beide "im Tandem", d.h. gleichzeitig, aufgrund der deutlich abgesenkten Wirkstoff-Konzentration den Wirkstoff nur in reduzierter Konzentration abgeben.

### Beispiel 1: Duftstoffdispenser für Autos mit Druckblister

Zur Herstellung eines Duftstoffdispensers für Autos wird eine verschliessbare Spritzgussbox benötigt, in der sich ein auswechselbarer Duftstoffblister und ein in das Spritzgussteil integrierter Druckblister zur Temperatur bedingten Schliessung des Duftstoffdispensors befinden.

Die Spritzgussbox ist so konzeptiert, dass sich durch eine Drehung des Spritzgussdeckels zwei einander gegenüberliegende Schlitze des Duftstoffdispensors (unten und oben) öffnen. Durch die geöffneten Schlitze kann die Umgebungsluft zwischen dem Duftstoff- und Druckblister hindurchströmen, den Duftstoff aufnehmen, der dann durch die konvektive, im Auto vorherrschende Luftströmung, verteilt wird.

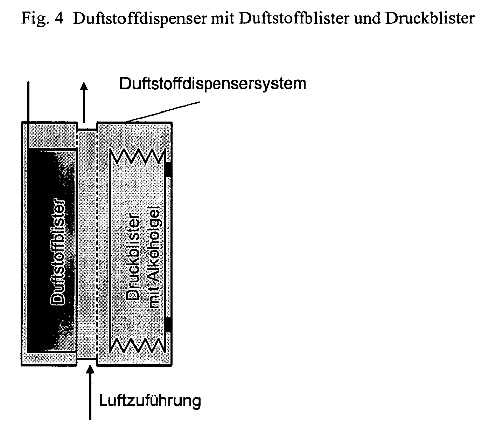

Bei zunehmender thermischer Belastung (z.B. bei starker Sonneneinstrahlung) erhöht sich der Dampfdruck im Druckblister, der sich in Folge in Richtung des Duftstoffblisters bis zu dessen Schliessung ausdehnt. Beim Absinken der Temperatur zieht sich auch der Druckblister wieder zurück und öffnet so erneut den Luftkanal (siehe Fig. 4).

### Herstellung des Duftstoffblisters:

Die den Duftstoff beinhaltende Polymermasse enthält folgende Ingredienzien:

| **Bestandteil** | **Funktion** | **Anteil (Gew,-%)** |
|---|---|---|
| Hydroxycitronellal | Duftstoff | 24 % |
| Pluronic PE 9400 | Blockpolymerisat (BASF) mit hoher Schmelzenthalpie | 21 % |
| Pluronic PE 10500 | Blockpolymerisat (BASF) mit hoher Schmelzenthalpie | 10 % |
| Methanol | Lösungsmittel | 5 % |
| Kaliumnitrat | Niedermolekulare, wasserlösliche Substanz mit endothermer Lösungs- bzw. Sorptionsenthalpie | 1 % |
| Gelatine | Stabilisierende Polypeptide, das sich bei mehr als 100°C zersetzt; bzw. Verdicker u. Gelierungsmittel | 4 % |
| Glutaraldehyd | Vernetzer | 0.5 % |
| Methylparaben | Konservierungsmittel | 0.5 % |
| Wasser | Lösungsmittel | 34 % |

Ein Polypropylenfaservlies mit einem Quadratmetergewicht von 180 g wird beidseitig mit dieser den Duftstoff enthaltenden Polymermasse beschichtet. Das beschichtete Vlies wird den Ausmassen des Blisters entsprechend in 40 x 40 mm Stücke gestanzt, in einen Blistersack eingebettet und verschweisst. Die Verschlussfolie wird unmittelbar vor dem Blistergebrauch abgezogen und der Blister in das Spritzgussteil eingeschoben.

### Herstellung des Druckblisters

Das bei thermischer Belastung den Druck erzeugende Alkoholgel besteht aus folgenden Ingredienzien:

| **Bestandteil** | **Anteil** | **Einheit** |
|---|---|---|
| Methanol | 890 | g/kg |
| Wasser | 100 | g/kg |
| Methocell | 10 | g/kg |

Ein zylindrischer aus Kunststoff bestehender Faltenbalg wird mit 3 g dieses Alkoholgels beschickt und dicht verschlossen (verschweisst). Die Fixierung des Druckblisters erfolgt auf der zum Duftstoffblister abgekehrten Seite, durch punktuelle Verschweissung mit dem Rand des Duftstoffdispensers (siehe Fig. 4).

Der beschriebene Duftstoffdispenser zeichnet sich durch eine sehr gute Wärmestabilisierung bei 30 - 35 °C aus. Der Druckblister ist in der Lage, bei ca. 35 - 40 °C innerhalb von 6 Minuten den Luftkanal zwischen Duftstoff- und Druckblister (ca. 5 mm) zu schliessen. Durch diese Massnahme ist eine weitere Duftstoffabgabe unter erhöhten Temperaturen weitgehend verhindert.

### Beispiel 2: Duftstoffdispenser für den Krankenhausgebrauch ohne Druckblister

Der Einsatz des hier beschriebenen Duftstoffdispensers ist dann angebracht, wenn parallel zur "Wellness" verbreitenden Funktion auch eine den Patienten sedierende Wirkung erzielt werden soll. Dies ist z.B. dann der Fall, wenn ein Patient ein agitives, aber auch ein gegenüber dem Betreuungspersonal oder den Mitpatienten agressives Verhalten zeigt. Die hier eingesetzten Duftstoffdispenser unterscheiden sich von dem im Beispiel 1 beschriebenen durch das Fehlen des Druckblisters und die Zusammensetzung der Duftstoffspeichermasse und den damit verbundenen Funktionen des Duftstoff blisters.

### Herstellung der Duftstoffspeichermasse zur Beschichtung des Faservlieses.

30 g in Wasser verquollenes Glukan wird in eine Ethanollösung eingerührt, die das Beruhigungsmittel Happybelle PE der Fa. Mibelle AG in einer Konzentration von 50 g/l enthält. Nach zweistündiger Verweildauer des Glukans in der ethanolischen "Happybelle PE"-Lösung wird das Glukan zentrifugiert und in einem geschlossenen Gefäss aufbewahrt, bis zu dessen Einmischung in die den Duftstoff enthaltende Polymermasse. Die Polymermasse besteht aus 15 % (bezogen auf die gesamte Duftstoffspeichermasse) eines Fliederöls, 25 % Pluronic PE 10500 (BASF), 25 % Pluronic PE 9400 (BASF), und 5 % Methanol. Diese Mischung zeigt einen Schmelzbereich von 30 - 40°C.

Die erwähnten 15 % des Duftstoffes werden mit der geschmolzenen Polymermasse vermischt und homogenisiert. Anschliessend wird das das Beruhigungsmittel enthaltende Glukan in die Polymerschmelze eingerührt. Nach erfolgter Dispergierung wird das Polymergemisch in eine wässrige Gelatinelösung eingemischt, bestehend aus 150 g/l Gelatine, 20 g/l Dinatriumhydrogenphosphat und 10 g/l eines Konservierungsmittels. Das Mischungsverhältnis zwischen der Duftstoff enthaltenden Polymerlösung und der Gelatinelösung beträgt 70 % zu 30 %.

Eine typische Rezeptur für einen Krankenhausblister ist in der nachfolgenden Tabelle gezeigt:

| **Bestandteil** | **Funktion** | **Anteil (Gew.-%)** |
|---|---|---|
| Glukan+Happybelle | Mikrokapsel+"Wellnessstoff" | 21 % |
| Pluronic PE 9400 | Blockpolymerisat (BASF) mit hoher Schmelzenthalpie | 17.5% |
| Pluronic PE 10500 | Blockpolymerisat (BASF) mit hoher Schmelzenthalpie | 17.5% |
| Methanol | Lösungsmittel | 3.5% |
| Fliederöl | Duftstoff | 10.5% |
| Dinatriumphosphat | Niedermolekulare, wasserlösliche Substanz mit endothermer Lösungs- bzw. Sorptionsenthalpie | 0.6% |
| Gelatine | Stabilisierendes Polypeptide als Verdicker u. Gelierungsmittel | 4.5% |
| Germaben II-E | Konservierungsmittel (Diazolidinylhamstoff+ Methyl- und Propylparaben, Firma ISP) | 0.3% |
| Wasser | Lösungsmittel | 24.6 % |

Die so hergestellte Dispersionsmasse wird bei ca. 30 - 35 °C auf das Faservlies appliziert.
Das beschichtete Faservlies wird anschliessend gestanzt und in der Blisterbox verschweisst. Die Benützung dieses Blisters ist analog der in Beispiel 1 beschriebenen, mit dem Unterschied, dass zwei Funktionen, eine "Wellness" verbreitende und eine sedierende, erfüllt werden.

Die so hergestellten Duftstoffdispensersysteme zeichnen sich durch eine gleichmässige, auch bei schwankenden Zimmertemperaturen nur wenig variierende Stoffabgabe aus. Der grosse Vorteil dieser Systeme ist deren Mehrfachfunktion, wobei die vom Patienten wahrgenommene nur die "Wellness"-Funktion ist.

### Beispiel 3: Absorptionsblister für Arztpraxen, Taxis etc.

Der Einsatzbereich des folgend beschriebenen Blisters ist dann angebracht wenn in der Luft befindlich Bakterien adsorbiert und zerstört werden müssen. Dies ist typischer Weise dann der Fall, wenn infektiöse Krankheiten durch in der Luft befindliche Bakterien verbreitet werden, wie dies in Arztpraxen, öffentlichen Gebäuden, Krankenhäusern aber auch in Taxen der Fall ist. Der Unterschied zu den vorher beschriebenen Systemen liegt in der Anwendung bakteriensorbierender partikulär vorliegender Polymerer in Kombination mit Enzymen, welche die Bakterienzellwand zerstören.

### Herstellung des Absorptionsblisters

Als Trägermaterial wird ein 5 mm offenporiges Polyetherschaummaterial verwendet (Polinazell PPI 45, Fritz Nauer AG, Schweiz), welches mit der nachfolgend beschriebenen Masse beschichtet wird. Das Trägermaterial wird mit 0.15 g/cm² der Beschichtungsmasse beaufschlagt.

Die Herstellung der Beschichtungsmasse erfolgt, indem das Bindemittel, der Dispergator und das Hydrogel sequentiell in eine Wasservorlage von 800 ml eingerührt werden. Anschliessend erfolgt die Zugabe und Einmischung der in 50 ml Wasser aufgeschlämmten Enzyme. Die mit Quab 188 modifizierten Cellulosemikropartikel (ca. 5 µm) werden in 100 ml Wasser aufgeschlämmt und dem enzymhaltigen Ansatz beigemischt. Abschliessend wird der Ansatz mit Wasser auf ein kg aufgefüllt. Die nun fertig gestellte Beschichtungsmasse wird mittels eines Rakelsystems auf den Schaumstoffträger appliziert. Der Beschichtungsgrad ist so gewählt, dass ca. 30 % des Porenvolumens des Trägermaterials nach wie vor offen sind. In einem anschliessenden Infrarot-Kanal wird ca. 20 % des Wassergehaltes verdampft, um so eine feste Hydrogelmasse zu erhalten. Die benötigten Blistereinheiten können in der gewünschten Grösse ausgestanzt und gerahmt werden.

| **Bestandteil** | **Funktion** | **Anteil %** |
|---|---|---|
| | | |
| Carbopol EZ-2 | Bindemittel (wirkt auch als Verdicker) | 0.5 |
| Dapral GE 202 | Dispergator | 1.2 |
| Borchi Gel L75N | Hydrogel (Verdicker) | 0.5 |
| α-Amylase | Enzym | 0.2 |
| Lysozym | Enzym | 0.2 |
| Lipase | Enzym | 0.2 |
| Mikrocellulose, Quab 188 derivatisiert | Bakterienadsorptionsmittel | 0.5 |
| Wasser | Lösungsmittel | 96.7 |

"Carbopol EZ-2" ist eine vernetzte Polyacrylsäure, die als Bindemittel und als Verdicker wirkt. "Dapral GE 202" ist ein Copolymer aus ethoxylieter Maleinsäure und and 1-Dodecen. "Borchi Gel L75N" beeinflusst das rheologische Verhalten und stellt ein in hydrophober Weise modifiziertes ethoxyliertes Urethan dar, das zur Selbstassoziation neigt und zusammen mit grenzflächenaktiven Mitteln die Viskosität beeinflusst. "Quab 188" (Handelsname von Degussa) bedeutet eine Derivatisierung mit 3-Chlor-2-hydroxypropyltrimethyl-ammoniumchlorid.

## Patentansprüche

1. Blistersystem, umfassend
- mindestens einen ersten Blister zur Abgabe eines Wirkstoffes (= Donorblister),
wobei dieser Blister umfasst:
c) mindestens eine Matrix, und
d) mindestens einen Wirkstoff, und
e) mindestens ein Polymer, dessen Schmelz- oder Zersetzungstemperatur höher als 100 °C liegt, und
f) mindestens ein Polymer, das bei einer Temperatur von 23 - 40 °C schmilzt; und
- mindestens einen zweiten Blister (= Akzeptorblister), wobei dieser Blister umfasst:
c) mindestens eine Matrix, und
h) mindestens ein Kunstharz, und / oder
i) mindestens einen Dispergator, und
j) mindestens ein Krankheitserreger absorbierendes oder abtötendes oder hemmendes Mittel, und / oder mindestens ein Mikropartikel und/oder gasförmige Substanzen absorbierendes Mittel, und
k) mindestens ein hydrolytisch wirkendes Enzym, und
l) mindestens einen partikulären Träger.

2. Blistersystem mit Donorblister zur Abgabe eines Wirkstoffes nach Anspruch 1 mit
g) einer niedermolekularen wasserlöslichen Substanz mit endothermer Lösungs- bzw. Sorptionsenthalpie.

3. Blistersystem nach Anspruch 2, wobei die niedermolekulare wasserlösliche Substanz g) Harnstoff, sekundäres Natriumphosphat und/oder Kaliumnitrat enthält.

4. Blistersystem nach einem der Ansprüche 1, 2 oder 3, wobei die Matrix c) eine faser- oder schaumstoffartige Matrix ist.

5. Blistersystem nach einem der Ansprüche 1, 2, 3 oder 4, wobei die Matrix c) ein Faservlies aus natürlichen und/oder synthetischen Fasern, insbesondere ein Polypropylenvlies, oder einen Schaumstoff, insbesondere ein Polyether-, Polyester-oder Polyamid-Schaumstoff mit offenen Poren, ist.

6. Blistersystem nach einem der Ansprüche 1 bis 5, wobei der Wirkstoff d) und / oder das Mittel j) inkapsuliert ist, und das Inkapsulierungsmittel vorzugsweise Cyclodextrin, Glukan, Chitosan und/oder Dendrimere, sowie Silikatpartikel, vorzugsweise Zeolithe und Kaoline, umfaßt.

7. Blistersystem nach einem der Ansprüche 1 bis 6, wobei der Wirkstoff d) und / oder das Mittel j) als Vesikel, Liposom und/oder Mikrokapsel formuliert ist.

8. Blistersystem nach einem der Ansprüche 1 bis 7, wobei das Polymere e) mindestens ein vernetzbares Polypeptid, Polysaccharid, Polyvinylalkohol, und/oder Blockpolymerisat auf der Basis von Polyvinylalkohol und/oder Polyvinylacetat / Polyvinylpyrrolidon enthält.

9. Blistersystem nach einem der Ansprüche 1 bis 8, wobei das Polymere f) mit dem Polymeren e) und/oder dem Wirkstoff d) eine homogene Phase, eine wässrige Dispersion oder eine Emulsion bildet.

10. Blistersystem nach einem der Ansprüche 1 bis 9, wobei das Polymere f) ein Ethylen / Propylenoxid - Blockpolymerisat, ein Fettsäurewachs, und/oder ein Polyethylenwachs enthält.

11. Blistersystem nach einem der Ansprüche 1 bis 10, wobei das Polymere f) Pluronic PE 9400, und / oder Pluronic PE 10500 (BASF) enthält.

12. Blistersystem nach einem der Ansprüche 1 bis 11, wobei das Mittel j) umfasst:
Silber enthaltende Mikro- und/oder Nanopartikel, polymere quaternäre Ammonium-Verbindungen, und/oder Mikro- und/oder Nanopartikel auf der Basis von Glucan, Microcellulose oder polymere Kieselsäure, welche mit quaternären Ammonium-Verbindungen modifiziert sind.

13. Blistersystem nach einem der Ansprüche 1 bis 12, wobei das hydrolytisch wirkende Enzym k) Lysozym, Amylasen, insbesondere alpha-Amylase, Cellulase, Glucanase, Pektinase, und Lipase umfasst.

14. Blistersystem nach einem der Ansprüche 1 bis 13, wobei der Donor- bzw. Akzeptorblister
- mindestens ein Lösungsmittel, und/oder
- mindestens ein Verdickungs- und/oder Geliermittel, und/oder
- mindestens ein Vernetzungsmittel, und/oder
- mindestens ein Konservierungsmittel, und/oder
- mindestens einen grenzflächenaktiven Stoff enthält.

15. Blistersystem nach einem der Ansprüche 1 bis 14,
welches zusätzlich mindestens einen Druckblister umfaßt,
wobei der Druckblister
a) ein in mindestens einer Dimension ausdehnungsfähiges Behältnis aufweist, das
b) mindestens ein niedrig siedendes Lösungsmittel enthält.

16. Druckblister nach Anspruch 15, wobei
das Lösungsmittel b) ein Verdickungsmittel und/oder ein Sorptionsmittel enthält.

17. Druckblister nach Anspruch 16, wobei
- das Verdickungsmittel ein Cellulosederivat und/oder Polyvinylpyrrolidon, und/oder wobei
- das Sorptionsmittel chemisch modifizierte Siliciumdioxid-Nanopartikel umfasst.

18. Druckblister nach Anspruch 17, wobei das Verdickungsmittel eine Hydroxypropyl-Methylcellulose und/oder eine Ethylhydroxyethyl-Cellulose ist.

19. Verwendung des Stoffdispensersystems nach einem der Ansprüche 1 bis 18 zur Abgabe von Wirkstoffen.
